(19) Europäisches Patentamt
European Patent Office
Office européen des brevets

(11) **EP 4 692 793 A1**

(12) **EUROPEAN PATENT APPLICATION**
published in accordance with Art. 153(4) EPC

(43) Date of publication:
**11.02.2026 Bulletin 2026/07**

(21) Application number: **24780769.6**

(22) Date of filing: **29.03.2024**

(51) International Patent Classification (IPC):
***G01N 33/48*** (2006.01)

(52) Cooperative Patent Classification (CPC):
**G01N 33/48**

(86) International application number:
**PCT/JP2024/012963**

(87) International publication number:
**WO 2024/204674 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053035**

(71) Applicant: Sekisui Medical Co., Ltd.
**Tokyo 103-0027 (JP)**

(72) Inventors:
- **HOSSAIN, Md Shahadat**
  **Tokyo 103-0027 (JP)**
- **KOMAI, Kuniya**
  **Tokyo 103-0027 (JP)**
- **UCHIYAMA, Takaya**
  **Tokyo 103-0027 (JP)**
- **INOUE, Tomonori**
  **Tokyo 103-0027 (JP)**

(74) Representative: **Ter Meer Steinmeister & Partner Patentanwälte mbB**
**Nymphenburger Straße 4**
**80335 München (DE)**

(54) **BLOOD COLLECTION CONTAINER, AND METHOD FOR SEPARATING MONONUCLEAR CELLS**

(57)    Provided is a blood collection container capable of increasing a recovery amount of mononuclear cells. A blood collection container includes a blood collection container main body; a specific gravity liquid contained in the blood collection container main body; and a blood separation material contained in the blood collection container main body, in which the specific gravity liquid contains an amino acid.

[FIG. 1.]

**Description**

**TECHNICAL FIELD**

The present invention relates to a blood collection container. The present invention also relates to a method for separating mononuclear cells using the blood collection container.

**BACKGROUND ART**

In clinical examination, a blood collection container such as a blood collection tube is widely used to collect blood. As an example of the blood collection container, a blood collection container capable of separating mononuclear cells from blood is also known.

For example, Patent Document 1 below discloses a method for separating mononuclear cells from blood by performing the following steps (a) to (d). (a) A container including a closed end and an open end is provided. (b) A gel-like substance and a water-soluble density gradient substance having a specific gravity larger than that of the substance are placed in the container. (c) A liquid sample is placed in the container. (d) The container is placed under centrifugal force to separate the liquid sample into a heavy phase and a light phase, and a barrier layer is established between the heavy phase and the light phase, the barrier layer consisting of separate layers of the gel-like substance and the water-soluble density gradient substance.

**Related Art Document**

**Patent Document**

Patent Document 1: JP S61-84557 A

**SUMMARY OF THE INVENTION**

**PROBLEMS TO BE SOLVED BY THE INVENTION**

In order to separate mononuclear cells from blood, a blood collection container including a specific gravity liquid may be used. In this blood collection container, mononuclear cells and blood cell components other than mononuclear cells can be separated by centrifuging the blood collection container from which the blood is collected.

Incidentally, a sterilized blood collection container may be used. For example, a blood collection container used as a medical device product needs to be sterilized. However, it is difficult to increase the recovery amount of mononuclear cells in the sterilized blood collection container in the related art.

An object of the present invention is to provide a blood collection container capable of increasing the recovery amount of the mononuclear cells. Another object of the present invention is to provide a method for separating mononuclear cells using the blood collection container.

**MEANS FOR SOLVING THE PROBLEMS**

In the present specification, the following blood collection container and method for separating mononuclear cells are disclosed.

Item 1. A blood collection container including: a blood collection container main body; a specific gravity liquid contained in the blood collection container main body; and a blood separation material contained in the blood collection container main body, in which the specific gravity liquid contains an amino acid.

Item 2. The blood collection container according to item 1, in which the amino acid contains a basic amino acid or a neutral amino acid.

Item 3. The blood collection container according to item 1 or 2, in which the amino acid contains β-alanine, proline, histidine, or glycine.

Item 4. The blood collection container according to any one of items 1 to 3, in which the amino acid contains β-alanine.

Item 5. The blood collection container according to any one of items 1 to 4, in which a content of the amino acid is 0.01 wt% or more and 5 wt% or less in 100 wt% of the specific gravity liquid.

Item 6. The blood collection container according to any one of items 1 to 5, in which the specific gravity liquid contains a component (X) that is solid at 25°C different from the amino acid.

Item 7. The blood collection container according to item 6, in which the component (X) contains polysucrose.

Item 8. The blood collection container according to item 6, in which the component (X) contains sodium diatrizoate.

Item 9. The blood collection container according to item 6, in which the component (X) contains polysucrose and sodium diatrizoate.

Item 10. The blood collection container according to any one of items 6 to 9, in which the component (X) contains silica fine particles.

Item 11. The blood collection container according to any one of items 6 to 10, in which a weight ratio of the content of the amino acid to the content of the component (X) in the specific gravity liquid is 0.001 or more and 0.5 or less.

Item 12. The blood collection container according to any one of items 1 to 11, in which the blood separation material is a composition for blood separation.

Item 13. The blood collection container according to item 12, in which the composition for blood separation contains an organic component having fluidity at 25°C and an inorganic fine powder, the organic component contains a resin, and the inorganic fine powder contains fine powder silica.

Item 14. The blood collection container according to any one of items 1 to 13, including an anticoagulant contained in the blood collection container main body.

Item 15. The blood collection container according to any one of items 1 to 14, in which the blood collection container main body is a polyethylene terephthalate container.

Item 16. The blood collection container according to any one of items 1 to 15, which is a blood collection container sterilized with γ rays.

Item 17. A method for separating mononuclear cells, including the steps of: collecting blood into the blood collection container according to any one of items 1 to 16; and centrifuging the blood collection container from which the blood has been collected.

## EFFECT OF THE INVENTION

A blood collection container according to the present invention includes a blood collection container main body, a specific gravity liquid contained in the blood collection container main body, and a blood separation material contained in the blood collection container main body, in which the specific gravity liquid contains an amino acid. Since the blood collection container according to the present invention has the above-described configuration, the recovery amount of the mononuclear cells can be increased.

## BRIEF DESCRIPTION OF DRAWINGS

[Fig. 1] Fig. 1 is a front sectional view schematically illustrating a blood collection container according to a first embodiment of the present invention.
[Fig. 2] Fig. 2 is a front sectional view schematically illustrating a blood collection container according to a second embodiment of the present invention.
[Fig. 3] Fig. 3 is a front sectional view schematically illustrating a blood collection container according to a third embodiment of the present invention.

## MODE FOR CARRYING OUT THE INVENTION

Hereinafter, the present invention will be described in detail.

A blood collection container according to the present invention includes a blood collection container main body, a specific gravity liquid contained in the blood collection container main body, and a blood separation material contained in the blood collection container main body, in which the specific gravity liquid contains an amino acid.

Since the blood collection container according to the present invention has the above-described configuration, the recovery amount of the mononuclear cells can be increased. In the blood collection container according to the present invention, since the specific gravity liquid contains the amino acid, the recovery amount of the mononuclear cells can be increased as compared with the blood collection container in the related art in which the specific gravity liquid does not contain amino acids.

As the blood collection container, a sterilized blood collection container may be used. For example, a blood collection container used as a medical device product needs to be sterilized. The present inventors have found that it is difficult to increase the recovery amount of the mononuclear cells in the sterilized blood collection container, and it is particularly difficult to increase the recovery amount of mononuclear cells in the sterilized blood collection container with γ rays. The present inventors have extensively conducted studies, and found that 1) the reason why it is difficult to increase the recovery amount of the mononuclear cells using the blood collection container in the related art is that precipitates form in the specific gravity liquid during the sterilization treatment, causing the properties of the specific gravity liquid to change, and 2) by using a specific gravity liquid containing an amino acid, precipitates are less likely to be generated in the specific gravity liquid. Incidentally, the reason why precipitates are hardly generated in the specific gravity liquid by using the specific gravity liquid containing the amino acid is presumed to be because radicals generated during the sterilization treatment are inactivated by the amino acid, but the present invention is not limited thereto. In the blood collection container according to the present invention, generation of the precipitates in the specific gravity liquid can be suppressed, and a change in the properties of the specific gravity liquid can be effectively suppressed, so that the recovery amount of the mononuclear cells can be increased even after sterilization.

In addition, in the blood collection container according to the present invention, the mononuclear cells can be easily separated from blood. In the blood collection container according to the present invention, for example, the mononuclear cells can be separated by collecting blood in the blood collection container and then centrifuging the blood collection container. In the blood collection container according to the present invention, by appropriately adjusting the specific gravity of the specific gravity liquid, the mononuclear cells can be suspended in a specific gravity liquid layer or a layer containing the mononuclear cells can be formed above the specific gravity liquid layer after centrifugation.

Hereinafter, details and the like of the blood collection container according to the present invention will be described. In the following description, "(meth)acryl" means one or both of "acryl" and "methacryl".

(Specific gravity liquid)

The blood collection container includes a specific gravity liquid contained in the blood collection container main body. The specific gravity liquid may be referred to as a density gradient liquid. The specific gravity liquid contains an amino acid. The specific gravity liquid preferably contains a component (X) that is solid at 25°C and is different from amino acids. The specific gravity liquid preferably contains water.

<Amino acid>

The specific gravity liquid contains an amino acid. The above amino acid may be used alone, or two or more kinds thereof may be used in combination.

Examples of the amino acid include basic amino acids such as histidine and arginine; neutral amino acids such as $\alpha$-alanine, $\beta$-alanine, proline, glycine, cysteine, serine, leucine, isoleucine, and phenylalanine; and acidic amino acids such as glutamic acid.

The amino acid preferably contains a basic amino acid or a neutral amino acid, more preferably contains $\beta$-alanine, proline, histidine, or glycine, further preferably contains $\beta$-alanine, and is particularly preferably $\beta$-alanine. In this case, precipitates are further less likely to occur in the specific gravity liquid during the sterilization treatment of the blood collection container, and thus the recovery amount of the mononuclear cells can be further increased.

In 100 wt% of the specific gravity liquid, the content of the amino acid is preferably 0.001 wt% or more, more preferably 0.01 wt% or more, and still more preferably 0.1 wt% or more, and preferably 10 wt% or less, more preferably 8 wt% or less, still more preferably 5 wt% or less, even more preferably 3 wt% or less, and particularly preferably 2 wt% or less. When the content of the amino acid is the above lower limit or more and the above upper limit or less, precipitates are further less likely to be generated in the specific gravity liquid during the sterilization treatment of the blood collection container, and thus the recovery amount of the mononuclear cells can be further increased. The content of the amino acid in 100 wt% of the specific gravity liquid may be 2 wt% or more or 3 wt% or more.

In the specific gravity liquid, the weight ratio of the content of the amino acid to the content of the component (X) (content of amino acid/content of component (X)) is preferably 0.001 or more, more preferably 0.005 or more, and still more preferably 0.01 or more, and preferably 10 or less, more preferably 5 or less, still more preferably 1 or less, even more preferably 0.5 or less, and particularly preferably 0.3 or less. When the weight ratio (content of amino acid/content of component (X)) is the above lower limit or more and the above upper limit or less, precipitates are further less likely to be generated in the specific gravity liquid during the sterilization treatment of the blood collection container, and thus the recovery amount of the mononuclear cells can be further increased.

<Component (X) that is solid at 25°C different from amino acid>

The specific gravity liquid preferably contains the component (X). The component (X) is a component that is solid at 25°C and is different from the amino acid. The component (X) is different from the amino acid. The component (X) is a component that is solid at 25°C. As the component (X), a known component in the related art (component that is solid at 25°C different from amino acid) used in a specific gravity liquid can be used. The above component (X) may be used alone, or two or more kinds thereof may be used in combination.

In the specific gravity liquid in the related art that contains the component (X), precipitates are generated in the specific gravity liquid during the sterilization treatment of the blood collection container, and the properties of the specific gravity liquid are likely to change. On the other hand, in the blood collection container according to the present invention, even when the specific gravity liquid contains the component (X), since the specific gravity liquid contains the amino acid, the precipitates are less likely to be generated in the specific gravity liquid. As a result, in the blood collection container according to the present invention, the recovery amount of the mononuclear cells can be increased even after the sterilization treatment.

Examples of the component (X) include polysucrose, sodium diatrizoate, sodium metrizoate, silica fine particles, sodium chloride, sodium ascorbate, hydroxyethyl starch, sucrose, dextran, and iodixanol.

The component (X) is preferably polysucrose, sodium diatrizoate, sodium metrizoate, silica fine particles, sodium chloride, sodium ascorbate, hydroxyethyl starch, sucrose, dextran, or iodixanol, and more preferably polysucrose, sodium diatrizoate, silica fine particles, or sodium chloride. In this case, the recovery amount of THE mononuclear cells can be further increased.

Polysucrose:

The component (X) preferably contains polysucrose. The specific gravity liquid preferably contains polysucrose. As the polysucrose, known polysucrose in the related art used in the specific gravity liquid can be used. In the specific gravity liquid containing polysucrose in the related art, precipitates are likely to be generated in the specific gravity liquid during the sterilization treatment of the blood collection container, but in the present invention, even when the specific gravity liquid contains polysucrose, the precipitates are less likely to be generated in the specific gravity liquid, and thus the recovery amount of the mononuclear cells can be further increased.

The number average molecular weight of the polysucrose is preferably 150,000 or more and more preferably 300,000 or more, and preferably 700,000 or less and more preferably 550,000 or less.

The number average molecular weight means a number average molecular weight in terms of pullulan measured by gel permeation chromatography (GPC).

The content of the polysucrose in 100 wt% of the specific gravity liquid is preferably 0.1 wt% or more, more preferably 1 wt% or more, still more preferably 5 wt% or more, and particularly preferably 8 wt% or more, and preferably 50 wt% or less, more preferably 30 wt% or less, and still more preferably 20 wt% or less. When the content of the polysucrose is the above lower limit or more and the above upper limit or less, the recovery amount of the mononuclear cells can be further increased.

In the specific gravity liquid, the weight ratio of the content of the amino acid to the content of the polysucrose (content of amino acid/content of polysucrose) is preferably 0.001 or more, more preferably 0.005 or more, and still more preferably 0.01 or more, and preferably 5 or less, more preferably 1 or less, still more preferably 0.5 or less, and particularly preferably 0.1 or less. When the weight ratio (content of amino acid/content of polysucrose) is the above lower limit or more and the above upper limit or less, the precipitates are further less likely to be generated in the specific gravity liquid during the sterilization treatment of the blood collection container, and thus the recovery amount of the mononuclear cells can be further increased.

In 100 wt% of the component (X), the content of the polysucrose is preferably 5 wt% or more, more preferably 15 wt% or more, still more preferably 25 wt% or more, and particularly preferably 40 wt% or more, and preferably 90 wt% or less, more preferably 80 wt% or less, and still more preferably 70 wt% or less. When the content of the polysucrose is the above lower limit or more and the above upper limit or less, the recovery amount of the mononuclear cells can be further increased.

Sodium diatrizoate:

The component (X) preferably contains sodium diatrizoate. The specific gravity liquid preferably contains sodium diatrizoate. In the known specific gravity liquid containing sodium diatrizoate in the related art, precipitates are likely to be generated in the specific gravity liquid during the sterilization treatment of the blood collection container, but in the present invention, even when the specific gravity liquid contains sodium diatrizoate, the precipitates are less likely to be generated in the specific gravity liquid, and thus the recovery amount of the mononuclear cells can be increased.

The content of the sodium diatrizoate in 100 wt% of the specific gravity liquid is preferably 0.5 wt% or more, more preferably 1 wt% or more, and still more preferably 5 wt% or more, and preferably 25 wt% or less, more preferably 20 wt% or less, and still more preferably 15 wt% or less. When the content of the sodium diatrizoate is the above lower limit or more and the above upper limit or less, the recovery amount of the mononuclear cells can be further increased.

In the specific gravity liquid, the weight ratio of the content of the amino acid to the content of the sodium diatrizoate (content of amino acid/content of sodium diatrizoate) is preferably 0.0001 or more, more preferably 0.001 or more, and still more preferably 0.005 or more, and preferably 5 or less, more preferably 1 or less, and still more preferably 0.5 or less. When the weight ratio (content of amino acid/content of sodium diatrizoate) is the above lower limit or more and the above upper limit or less, precipitates are further less likely to be generated in the specific gravity liquid during the sterilization treatment of the blood collection container, and thus the recovery amount of the mononuclear cells can be further increased.

In 100 wt% of the component (X), the content of the sodium diatrizoate is preferably 15 wt% or more, more preferably 25 wt% or more, and still more preferably 35 wt% or more, and preferably 95 wt% or less, more preferably 90 wt% or less, and still more preferably 85 wt% or less. When the content of the sodium diatrizoate is the above lower limit or more and the above upper limit or less, the recovery amount of the mononuclear cells can be further increased.

The component (X) more preferably contains polysucrose and sodium diatrizoate. In the specific gravity liquid containing these components in the related art, precipitates are likely to be generated in the specific gravity liquid during the sterilization treatment of the blood collection container, but in the present invention, even when the specific gravity liquid contains containing these components, the precipitates are less likely to be generated in the specific gravity liquid, and thus the recovery amount of the mononuclear cells can be further increased.

The total content of the polysucrose and the sodium diatrizoate in 100 wt% of the specific gravity liquid is preferably 1 wt% or more, more preferably 5 wt% or more, and still more preferably 10 wt% or more, and preferably 80 wt% or less, more preferably 60 wt% or less, and still more preferably 40 wt% or less. When the total content is the above lower limit or more and the above upper limit or less, the recovery amount of the mononuclear cells can be further increased.

In the specific gravity liquid, the weight ratio of the content of the amino acid to the total content of the polysucrose and the sodium diatrizoate (content of amino acid/total content of polysucrose and sodium diatrizoate) is preferably 0.001 or more, more preferably 0.005 or more, and still more preferably 0.01 or more, and preferably 10 or less, more preferably 5 or less, still more preferably 1 or less, even more preferably 0.5 or less, and particularly preferably 0.1 or less. When the weight ratio (content of amino acid/total content of polysucrose and sodium diatrizoate) is the above lower limit or more and the above upper limit or less, precipitates are further less likely to be generated in the specific gravity liquid during the sterilization treatment of the blood collection container, and thus the recovery amount of the mononuclear cells can be further increased.

In the specific gravity liquid, the content of the polysucrose is preferably larger than the content of the sodium diatrizoate. In this case, the recovery amount of the mononuclear cells can be further increased.

The total content of the polysucrose and the sodium diatrizoate in 100 wt% of the component (X) is preferably 80 wt% or more, more preferably 90 wt% or more, still more preferably 95 wt% or more, particularly preferably 99 wt% or more, and most preferably 100 wt%. When the total content is the above lower limit or more, the recovery amount of the mononuclear cells can be further increased. The total content of the polysucrose and the sodium diatrizoate in 100 wt% of the component (X) may be 100 wt% or less, less than 100 wt%, or 99 wt% or less.

Silica fine particles:

The component (X) preferably contains silica fine particles. As the silica fine particles, known silica fine particles in the related art used for the specific gravity liquid can be used. The silica fine particles are preferably silica fine particles coated with polyvinylpyrrolidone. As a suspension water solution of the silica fine particles coated with polyvinylpyrrolidone, Percoll (Cytiva) is commercially available and is easily available.

An average particle size of the silica fine particles is preferably 5 nm or more, more preferably 10 nm or more, and still more preferably 15 nm or more, and preferably 50 nm or less, more preferably 40 nm or less, and still more preferably 30 nm or less. The average particle size of the silica fine particles can be measured by electron microscopy.

The content of the silica fine particles in 100 wt% of the specific gravity liquid is preferably 0.1 wt% or more, more preferably 1 wt% or more, and still more preferably 10 wt% or more, and preferably 65 wt% or less, more preferably 45 wt% or less, and still more preferably 25 wt% or less. When the content of the silica fine particles is the above lower limit or more and the above upper limit or less, the recovery amount of the mononuclear cells can be further increased.

Sodium chloride:

The component (X) preferably contains sodium chloride. The specific gravity liquid preferably contains sodium chloride.

The content of the sodium chloride in 100 wt% of the specific gravity liquid is preferably 0.001 wt% or more, more preferably 0.01 wt% or more, and still more preferably 0.1 wt% or more, and preferably 15 wt% or less, more preferably 10 wt% or less, and still more preferably 5 wt% or less. When the content of sodium chloride is the above lower limit or more and the above upper limit or less, the recovery amount of the mononuclear cells can be further increased.

Sodium ascorbate:

The component (X) preferably contains sodium ascorbate. The specific gravity liquid preferably contains sodium ascorbate.

The content of the sodium ascorbate in 100 wt% of the specific gravity liquid is preferably 0.01 wt% or more and more preferably 0.1 wt% or more, and preferably 10 wt% or less and more preferably 5 wt% or less. When the content of the sodium ascorbate is the above lower limit or more and the above upper limit or less, the recovery amount of the mononuclear cells can be further increased.

<Water>

The specific gravity liquid preferably contains water. In the specific gravity liquid, water serves as a solvent. The water may be contained derived from a commercially available product containing the component (X).

The content of the water in 100 wt% of the specific gravity liquid is preferably 50 wt% or more, more preferably 60 wt% or more, and still more preferably 70 wt% or more, and particularly preferably 80 wt% or more, and preferably 99 wt% or less, more preferably 95 wt% or less, and still more preferably 90 wt% or less.

<Other components>

The specific gravity liquid may contain components other than the above-described components (amino acid, component (X), and water). Examples of the other components include glycerol. The above other components may be used alone, or two or more kinds thereof may be used in combination.

<Other details of specific gravity liquid>

The specific gravity of the specific gravity liquid at 25°C is preferably 1.060 or more, more preferably 1.065 or more, still more preferably 1.070 or more, and particularly preferably 1.080 or more, and preferably 1.098 or less, more preferably 1.095 or less, and still more preferably 1.090 or less. When the specific gravity of the specific gravity liquid at 25°C is the above lower limit or more and the above upper limit or less, the recovery amount of the mononuclear cells can be further increased.

The specific gravity of the specific gravity liquid at 25°C is measured using a densimeter (for example, "DA-130N" manufactured by Kyoto Electronics Manufacturing Co., Ltd.).

The specific gravity of the specific gravity liquid at 25°C is preferably larger than the specific gravity of the blood separation material at 25°C.

The osmotic pressure of the specific gravity liquid is preferably 285 mOsm/L or more, more preferably 300 mOsm/L or more, and still more preferably 310 mOsm/L or more, and preferably 420 mOsm/L or less, more preferably 400 mOsm/L or less, and still more preferably 390 mOsm/L or less. When the content of the osmotic pressure of the specific gravity liquid is the above lower limit or more and the above upper limit or less, the recovery amount of the mononuclear cells can be further increased.

The osmotic pressure of the specific gravity liquid is measured by a freezing point depression method using an osmotic pressure meter (for example, "OM-6060" manufactured by ARKRAY, Inc).

It is preferable that the specific gravity liquid is contained in the blood collection container main body on the bottom side from the blood separation material in the blood collection container main body. In the blood collection container, it is preferable that the blood separation material is located on the open end side of the blood collection container main body, and the specific gravity liquid is located on the bottom side of the blood collection container main body.

The amount of the specific gravity liquid contained in the blood collection container main body is preferably 0.1 mL or more, more preferably 0.15 mL or more, and still more preferably 0.2 mL or more, and preferably 0.4 mL or less, more preferably 0.35 mL or less, and still more preferably 0.3 mL or less with respect to 1 mL of the amount of blood collected in the blood collection container. In this case, the recovery amount of the mononuclear cells can be further increased.

The specific gravity liquid is preferably a specific gravity liquid having a property that no precipitate is generated when irradiated with γ rays at a dose of 30 kGy. Whether or not the precipitates have been generated can be visually confirmed.

(Blood separation material)

The blood collection container includes a blood separation material contained in the blood collection container main body. As the blood separation material, a known blood separation material in the related art can be used. Examples of the blood separation material include a composition for blood separation and a blood separation jig. The blood separation material is preferably the composition for blood separation because it is easy to prepare the blood separation material.

<Composition for blood separation>

The composition for blood separation is preferably a composition that moves between a specific gravity liquid layer and a blood cell layer during centrifugation to form a partition wall. In addition, the composition for blood separation is used for the purpose of preventing component migration between the specific gravity liquid layer and the blood cell layer after centrifugation. The composition for blood separation preferably has thixotropy.

As the composition for blood separation, a known composition for blood separation in the related art can be used.

The composition for blood separation preferably contains an organic component having fluidity at 25°C and an inorganic fine powder. One kind of each of the organic component having fluidity at 25°C and the inorganic fine powder may be used alone, and two or more kinds thereof may be used in combination.

Organic component having fluidity at 25°C:
The phrase "having fluidity at 25°C" means that the viscosity at 25°C is 500 Pa·s or less.

The viscosity of the organic component at 25°C is preferably 30 Pa·s or more and more preferably 50 Pa·s or more, and preferably 200 Pa·s or less and more preferably 100 Pa·s or less. When the viscosity is the above lower limit or more and the above upper limit or less, the fluidity of the composition for blood separation is enhanced, and the strength of the partition wall can be enhanced.

The viscosity of the organic component at 25°C is measured using an E-type viscometer (for example, "TVE-35" manufactured by TOKISANGYO) under the conditions of 25°C and a shear rate of 1.0 seconds $^{-1}$.

Examples of the organic component include a resin, and a mixture of a resin and an organic compound such as a plasticizer. Therefore, the organic component preferably contains the resin, and more preferably contains the resin and the organic compound. When the organic component is a mixture of the resin and the organic compound, the resin or the organic compound may not have fluidity as long as the mixture (organic component) has fluidity. When the organic component is a mixture of the resin and the organic compound, the resin may be, for example, a resin that is solid at 25°C. Only one kind of each of the resin and the organic compound may be used, or two or more kinds thereof may be used in combination.

Examples of the resin include a petroleum resin, a cyclopentadiene resin, a polyester resin, a polyurethane resin, a (meth)acrylic resin, a silicone resin, an α-olefin-fumaric acid ester copolymer, a copolymer of sebacic acid, 2,2-dimethyl-1,3-propanediol, and 1,2-propanediol, a polyether polyurethane resin, and a polyether polyester resin. The above resin may be used alone, or two or more kinds thereof may be used in combination.

The resin preferably contains a petroleum resin, a cyclopentadiene resin, a polyester resin, or a (meth)acrylic resin.

Examples of commercially available products of the petroleum resin include "Rigalite S5090" manufactured by Eastman Chemical Company.

Examples of the cyclopentadiene resin include a polymer of a cyclopentadiene monomer, a copolymer of a cyclopentadiene monomer and an aromatic monomer, and a dicyclopentadiene resin. The cyclopentadiene resin may be hydrogenated. The polymer of the cyclopentadiene monomer and the copolymer of the cyclopentadiene monomer and the aromatic monomer may be oligomers.

Examples of the cyclopentadiene monomer include cyclopentadiene, dicyclopentadiene, and alkyl-substituted derivatives of cyclopentadiene.

Examples of the aromatic monomer include styrene, methylstyrene, indene, and methylindene.

Examples of a commercially available product of the dicyclopentadiene resin include "SCOREZ SU500" and "SCOREZ SU90" manufactured by COLON CO.,LTD.

Examples of the polyester resin include a polyalkylene terephthalate resin and a polyalkylene naphthalate resin. Examples of the polyalkylene terephthalate resin include polyethylene terephthalate, polybutylene terephthalate, and poly-1,4-cyclohexanedimethylene terephthalate.

Examples of the polyurethane resin include a reaction product of a polyol compound and an isocyanate compound.

Examples of the (meth)acrylic resin include a resin obtained by polymerizing at least one (meth)acrylic acid ester monomer, and a resin obtained by polymerizing at least one (meth) acrylic acid ester monomer and at least one monomer other than the (meth)acrylic acid ester monomer.

Examples of the (meth)acrylic acid ester monomer include (meth)acrylic acid alkyl ester having an alkyl group having 1 to 20 carbon atoms, (meth)acrylic acid polyalkylene glycol ester, (meth)acrylic acid alkoxyalkyl ester, (meth) acrylic acid hydroxyalkyl ester, (meth)acrylic acid glycidyl ester, (meth)acrylic acid dialkylaminoalkyl ester, (meth)acrylic acid benzyl ester, (meth)acrylic acid phenoxyalkyl ester, (meth)acrylic acid cyclohexyl ester, (meth)acrylic acid isobornyl ester, and (meth)acrylic acid alkoxysilyl alkyl ester. The above (meth)acrylic acid ester monomer may be used alone, or two or more kinds thereof may be used in combination.

Examples of the organic compound include benzene polycarboxylic acid alkyl ester derivatives. The organic compound is preferably a benzene polycarboxylic acid alkyl ester derivative. Therefore, the organic component is preferably a mixture of the resin and the benzene polycarboxylic acid alkyl ester derivative.

Examples of the benzene polycarboxylic acid alkyl ester derivative include phthalic acid esters, trimellitic acid esters, and pyromellitic acid esters. The above benzene polycarboxylic acid alkyl ester derivative may be used alone, or two or more kinds thereof may be used in combination.

Examples of the trimellitic acid ester include tri-n-octyl trimellitic acid, triisooctyl trimellitic acid, and triisodecyl trimellitic acid.

Examples of the pyromellitic acid ester include tetraisooctyl pyromellitic acid.

Examples of commercially available products of the trimellitic acid ester include "Monocizer W700" and "Monocizer W-750" manufactured by DIC Corporation, and "Sansosizer TOTM" and "Sansocizer TITM" manufactured by New Japan Chemical Co., Ltd.

Examples of commercially available products of the pyromellitic acid ester include "Monocizer W-7010" manufactured by DIC Corporation.

The benzene polycarboxylic acid alkyl ester derivative is preferably a phthalic acid ester, a trimellitic acid ester, or a pyromellitic acid ester, and more preferably a trimellitic acid ester.

The content of the organic component in 100 wt% of the composition for blood separation is preferably 80 wt% or more and more preferably 85 wt% or more, and still more preferably 90 wt% or more, and preferably 97 wt% or less.

Inorganic fine powder:

Examples of the inorganic fine powder include fine powder silica, titanium oxide powder, calcium carbonate powder, zinc oxide powder, alumina powder, glass powder, talc powder, kaolin powder, bentonite powder, titania powder, and zirconium powder.

The inorganic fine powder is preferably fine powder silica, titanium oxide powder, calcium carbonate powder, zinc oxide powder, alumina powder, glass fine powder, talc powder, kaolin powder, bentonite powder, titania powder, or zirconium powder.

From the viewpoint of more effectively exhibiting the effect of the present invention, the inorganic fine powder preferably contains fine powder silica. When obtaining a composition for blood separation having a specific gravity at 25°C of 1.050 or more, the inorganic fine powder more preferably contains fine powder silica and an inorganic fine powder (second inorganic fine powder) other than the fine powder silica. However, even when the specific gravity of the composition for blood separation at 25°C is 1.050 or more, the inorganic fine powder may not contain the second inorganic fine powder. Even when the specific gravity of the composition for blood separation at 25°C is less than 1.050, the inorganic fine powder may contain the second inorganic fine powder. Each of the inorganic fine powder, the fine powder silica, and the second inorganic fine powder may be used alone, or two or more kinds thereof may be used in combination.

Examples of the fine powder silica include natural silica and synthetic silica. Examples of the synthetic silica include hydrophilic silica and hydrophobic silica. Hydrophilic silica has an action of imparting thixotropy to the composition for blood separation and adjusting the specific gravity, for example, by hydrogen bonding between hydroxyl groups on the particle surface. On the other hand, hydrophobic silica has a smaller thixotropic imparting effect than hydrophilic silica.

From the viewpoint of maintaining both the specific gravity and the thixotropy of the composition for blood separation in a suitable range, the fine powder silica preferably contains hydrophilic silica, and more preferably contains hydrophilic silica and hydrophobic silica. The fine powder silica preferably contains at least hydrophilic silica.

The second inorganic fine powder is preferably an inorganic fine powder having a specific gravity larger than that of fine powder silica, and more preferably an inorganic fine powder having a specific gravity of 3 or more, such as a zinc oxide powder, a titanium oxide powder, or an alumina powder.

The specific gravity of the second inorganic fine powder is preferably 3 or more, more preferably 3.5 or more, and still more preferably 4 or more. The specific gravity of the second inorganic fine powder is preferably as large as possible. When the specific gravity is the lower limit or more, the specific gravity of the composition for blood separation can be effectively increased.

The average particle size of the inorganic fine powder, the fine powder silica, and the second inorganic fine powder is not particularly limited. The average particle size of the inorganic fine powder, the fine powder silica, and the second inorganic fine powder may be 1 nm or more, 10 nm or more, 500 nm or less, or 100 nm or less.

The average particle size of the inorganic fine powder, the fine powder silica, and the second inorganic fine powder is an average diameter (volume average particle size) measured on a volume basis, and is a value of a median diameter (D50) of 50%. The volume average particle size (D50) can be measured by a laser diffraction/scattering method, an image analysis method, a Coulter method, a centrifugal sedimentation method, or the like. The volume average particle size (D50) is preferably determined by a laser diffraction/scattering method or an image analysis method.

The specific surface area of the fine powder silica is not particularly limited. The specific surface area of the fine powder silica may be 20 m$^2$/g or more, 100 m$^2$/g or more, 500 m$^2$/g or less, or 300 m$^2$/g or less.

The specific surface area of the fine powder silica is measured by a BET method.

The content of the hydrophilic silica in 100 wt% of the composition for blood separation is preferably 0.01 wt% or more, more preferably 0.10 wt% or more, and still more preferably 0.30 wt% or more, and preferably 2.50 wt% or less, and more preferably 2.00 wt% or less. When the content of the hydrophilic silica is the above lower limit or more and the above upper limit or less, both the specific gravity and the thixotropy of the composition for blood separation can be maintained in a more suitable range.

The content of the fine powder silica in 100 wt% of the composition for blood separation is preferably 0.1 wt% or more and more preferably 0.5 wt% or more, and preferably 10 wt% or less and more preferably 7 wt% or less. When the content of the fine powder silica is the above lower limit or more and the above upper limit or less, both the specific gravity and the thixotropy of the composition for blood separation can be maintained in a more suitable range.

The content of the second inorganic fine powder in 100 wt% of the composition for blood separation is preferably 0.01 wt% or more and more preferably 0.1 wt% or more, and preferably 10 wt% or less and more preferably 7 wt% or less. When the content of the second inorganic fine powder is the above lower limit or more and the above upper limit or less, the specific gravity of the composition for blood separation can be effectively increased.

The content of the inorganic fine powder in 100 wt% of the composition for blood separation is preferably 0.1 wt% or more and more preferably 0.5 wt% or more, and preferably 10 wt% or less and more preferably 7 wt% or less. When the content of the inorganic fine powder is the above lower limit or more and the above upper limit or less, the specific gravity of the composition for blood separation can be effectively increased.

Other components:

The composition for blood separation may contain components other than the above-described components as long as the effects of the present invention are not impaired. Examples of the other components include an organic gelling agent, a thermoplastic elastomer, a polyalkylene glycol, a silicone oil, an auxiliary solvent, an antioxidant, a colorant, and water. The above other components may be used alone, or two or more kinds thereof may be used in combination.

The specific gravity of the composition for blood separation at 25°C is preferably 1.060 or more, more preferably 1.065 or more, and still more preferably 1.070 or more, and preferably 1.095 or less, more preferably 1.090 or less, and still more preferably 1.085 or less. When the specific gravity of the composition for blood separation at 25°C is the above lower limit or more and the above upper limit or less, the effect of the present invention can be more effectively exhibited.

The specific gravity of the composition for blood separation at 25°C is measured by sequentially dropping 1 drop of the composition for blood separation into saline at 25°C whose specific gravity is adjusted stepwise at intervals of 0.002, and by flotation and sedimentation in saline.

The viscosity of the composition for blood separation at 25°C is preferably 100 Pa·s or more and more preferably 150 Pa·s or more, and preferably 500 Pa·s or less and more preferably 400 Pa·s or less. When the viscosity is the above lower limit or more and the above upper limit or less, the effect of the present invention can be more effectively exhibited.

The viscosity of the composition for blood separation at 25°C is measured using an E-type viscometer (for example, "TVE-35" manufactured by TOKISANGYO) under the conditions of 25°C and a shear rate of 1.0 seconds $^{-1}$.

<Blood separation jig>

The blood separation jig is a jig that moves between a specific gravity liquid layer and a blood cell layer during centrifugal separation to form a partition wall. In addition, the blood separation jig is used for the purpose of preventing component migration between the specific gravity liquid layer and the blood cell layer.

As the blood separation jig, a known jig in the related art can be used. Examples of the blood separation jig include a mechanical separator described in WO2010/132783A1 and the like.

Examples of the material of the blood separation jig include an elastomer.

(Anticoagulant)

The blood collection container preferably includes an anticoagulant contained in the blood collection container main body. As the anticoagulant, a known anticoagulant in the related art can be used. The above anticoagulant may be used alone, or two or more kinds thereof may be used in combination.

Examples of the anticoagulant include heparin, a metal salt of heparin, ethylenediaminetetraacetic acid (EDTA), a metal salt of EDTA, citric acid, and sodium citrate.

From the viewpoint of favorably exhibiting anticoagulation performance, the anticoagulant is preferably EDTA, a metal salt of EDTA, heparin, a metal salt of heparin, or sodium citrate.

The anticoagulant may be contained in a powder state or may be contained in a state of being dissolved in a liquid in the blood collection container main body. The anticoagulant may be present in both a powder state and a liquid state in the blood collection container main body.

Examples of the liquid include water and alcohol. The liquid is preferably water.

The anticoagulant is preferably disposed on an inner wall surface of the blood collection container main body or disposed on the surface of the blood separation material. The anticoagulant may be disposed on the inner wall surface of the blood collection container main body, may be disposed on the surface of the blood separation material, or may be disposed on both the inner wall surface of the blood collection container main body and the surface of the blood separation material.

When the anticoagulant is contained in a powder state, it is preferable that the anticoagulant in the powder state is attached on the inner wall surface of the blood collection container main body or disposed on the surface of the blood separation material. For example, the mixed solution of the anticoagulant and water is spray-coated on the inner wall surface of the blood collection container main body and dried, whereby the anticoagulant can be attached in the powder state on the inner wall surface of the blood collection container main body.

When the anticoagulant is contained in a state of being dissolved in a liquid, a mixed liquid of the anticoagulant and the liquid (liquid containing the anticoagulant) is preferably disposed on the surface of the blood separation material.

The amount of the anticoagulant contained in the blood collection container main body is not particularly limited as long as the anticoagulant performance can be exhibited.

When the blood collected in the blood collection container is blood to which an anticoagulant is added, the blood collection container may not include an anticoagulant.

(Blood collection container main body)

The shape of the blood collection container main body is not particularly limited. The blood collection container main body is preferably a bottomed tubular container. The blood collection container main body preferably has an open end at one end and a closed bottom portion (closed end) at the other end.

The material of the blood collection container main body is not particularly limited. Examples of the material of the blood collection container main body include thermoplastic resins such as polyethylene, polypropylene, polystyrene, polyethylene terephthalate, polymethyl methacrylate, and polyacrylonitrile; thermosetting resins such as an unsaturated polyester resin, an epoxy resin, and an epoxy-acrylate resin; modified natural resins such as cellulose acetate, cellulose propionate, ethyl cellulose, and ethyl chitin; silicate glass such as soda lime glass, phosphosilicate glass, and borosilicate glass; and glass such as quartz glass. The materials of the blood collection container main body may be used alone, or two or more kinds thereof may be used in combination.

The blood collection container main body is preferably a resin container, more preferably a thermoplastic resin container, and still more preferably a polyethylene terephthalate container. When the blood collection container main body is a resin container (particularly, in the case of a polyethylene terephthalate container), a sterilization treatment method using γ rays is easily adopted as a sterilization treatment method of the blood collection container. In the blood collection container in the related art, when the sterilization treatment is performed with γ rays, a precipitate is generated in the specific gravity liquid, and the recovery amount of the mononuclear cells is likely to be reduced. However, in the present invention, since the generation of the precipitate in the specific gravity liquid can be effectively suppressed, the resin container can be used without any problem as the blood collection container main body. Therefore, when the blood collection container main body is a resin container (particularly, in the case of a polyethylene terephthalate container,), the effects of the present invention are more effectively exhibited.

(Plug)

The blood collection container preferably includes a plug. The plug is preferably attached to the open end of the blood collection container main body. As the plug, a known plug in the related art can be used. The plug is preferably formed of a material or has a shape that can be airtightly and liquid-tightly attached to the open end of the blood collection container main body. The plug is preferably configured such that a blood sampling needle can be inserted therethrough.

Examples of the plug include a plug having a shape fitted to the open end of the blood collection container main body, a sheet-like seal plug, and the like.

The plug may include a plug body such as a rubber plug and a cap member made of plastic or the like. In this case, it is possible to suppress the risk that the blood comes into contact with the human body when the plug is pulled out from the open end of the blood collection container main body after the blood collection.

Examples of the material of the plug (or the plug main body) include a synthetic resin, an elastomer, rubber, and metal foil. Examples of the rubber include butyl rubber and halogenated butyl rubber. Examples of the metal foil include aluminum foil. From the viewpoint of enhancing the sealing property, the material of the plug is preferably butyl rubber. The plug (or the plug body) is preferably a butyl rubber plug.

(Other details of blood collection container)

The blood collection container is suitably used for separating mononuclear cells from blood. The blood collection container is more suitably used for separating peripheral blood mononuclear cells (PBMC) from blood.

The amount of blood collected in the blood collection container is appropriately changed depending on the size, internal pressure, and the like of the blood collection container. The amount of blood collected in the blood collection container may be 1 mL or more, 2 mL or more, and 4 mL or more, and may be 12 mL or less, 11 mL or less, and 10 mL or less.

The blood collection container is preferably a blood collection tube. The blood collection container main body is preferably a blood collection tube main body.

The internal pressure of the blood collection container is not particularly limited. The internal pressure of the blood collection container is preferably reduced. When the blood collection container is a reduced pressure container, a predetermined amount of blood can be easily collected in the blood collection container. The blood collection container can also be used as a vacuum blood collection tube sealed by the plug after the inside is evacuated. In the case of the vacuum blood collection tube, a certain amount of blood can be easily collected regardless of the technical difference of the blood collector.

From the viewpoint of preventing bacterial infection, the inside of the blood collection container is preferably sterilized in accordance with the standard of ISO or JIS. As a method of the sterilization treatment, a known method in the related art can be used. Examples of the sterilization treatment method include $\gamma$-ray sterilization, electron beam sterilization, and high-pressure steam sterilization.

The blood collection container is preferably a sterilized blood collection container, and more preferably a blood collection container sterilized with $\gamma$ rays. In the blood collection container in the related art, when the sterilization treatment is performed with $\gamma$ rays, precipitates are more likely to be generated in the specific gravity liquid, and as a result, the recovery amount of the mononuclear cells is more likely to be reduced, but in the present invention, the generation of precipitates in the specific gravity liquid can be effectively suppressed even when the sterilization treatment is performed with $\gamma$ rays. Therefore, when the blood collection container is a blood collection container sterilized with $\gamma$ rays, the effect of the present invention is more effectively exhibited.

The blood collection container can be manufactured, for example, as follows.

The amino acid and the component (X) are dissolved in water to obtain a specific gravity liquid. The obtained specific gravity liquid is contained in the blood collection container main body. Next, the blood separation material is contained in the blood collection container main body. In addition, an anticoagulant is contained in the blood collection container main body.

Hereinafter, specific embodiments of the present invention will be described with reference to the drawings. In the following drawings, the size, thickness, shape, and the like may be different from the actual size, thickness, shape, and the like for convenience of illustration.

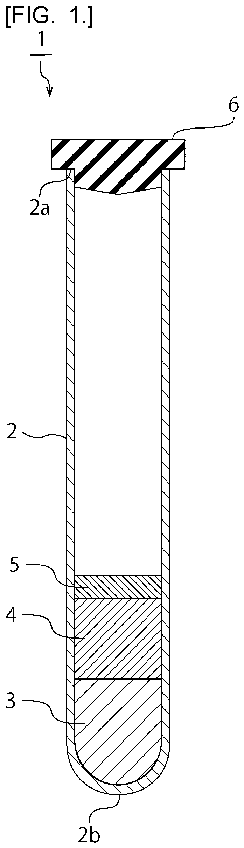

Fig. 1 is a front cross-sectional view schematically illustrating a blood collection container according to a first embodiment of the present invention.

A blood collection container 1 shown in Fig. 1 includes a blood collection container main body 2, a specific gravity liquid 3, a composition for blood separation 4, a liquid 5 containing an anticoagulant, and a plug 6. The blood collection container main body 2 has an open end 2a at one end and a bottom 2b at the other end. The plug 6 is attached to the open end 2a of the blood collection container main body 2.

The specific gravity liquid 3 is contained in the bottom 2b of the blood collection container main body 2. The specific gravity liquid 3 is contained in the blood collection container main body 2 closer to the bottom 2b than the composition for blood separation 4 in the blood collection container main body 2. The composition for blood separation 4 is contained in the blood collection container main body 2 closer to the open end 2a side than the specific gravity liquid 3 in the blood collection container main body 2. The liquid 5 containing the anticoagulant is disposed on the surface of the composition for blood separation 4. In the blood collection container 1, the specific gravity liquid 3, the composition for blood separation 4, and the liquid 5 containing the anticoagulant are arranged in this order from the bottom 2b side of the blood collection container main body 2 toward the open end 2a side.

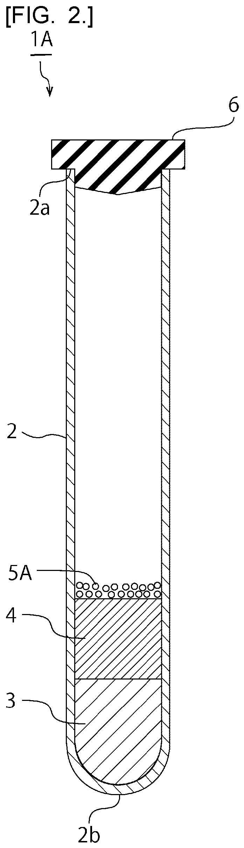

Fig. 2 is a front cross-sectional view schematically illustrating a blood collection container according to a second embodiment of the present invention.

A blood collection container 1A shown in Fig. 2 includes a blood collection container main body 2, a specific gravity liquid 3, a composition for blood separation 4, a powdery anticoagulant 5A, and a plug 6. The blood collection container 1 illustrated in Fig. 1 and the blood collection container 1A illustrated in Fig. 2 are different in the form of containing the anticoagulant. That is, in the blood collection container 1 illustrated in Fig. 1, the anticoagulant is contained in the blood collection container main body 2 in a state of being dissolved in a liquid, whereas in the blood collection container 1A illustrated in Fig. 2, the anticoagulant is contained in the blood collection container main body 2 in a powder state. More specifically, in the blood collection container 1A shown in Fig. 2, the powdery anticoagulant 5A is disposed on the surface of the composition for blood separation 4.

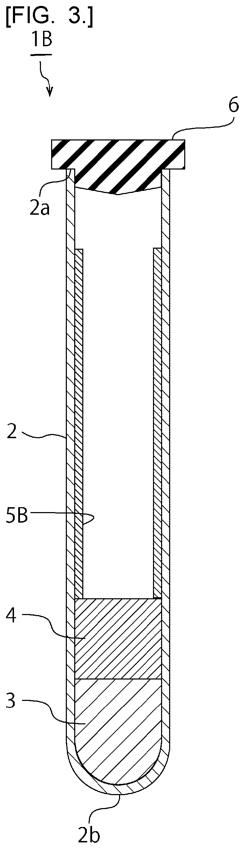

Fig. 3 is a front cross-sectional view schematically illustrating a blood collection container according to a third embodiment of the present invention.

A blood collection container 1B shown in Fig. 3 includes a blood collection container main body 2, a specific gravity liquid 3, a composition for blood separation 4, a powdery anticoagulant 5B, and a plug 6. The blood collection container 1 illustrated in Fig. 1 and the blood collection container 1B illustrated in Fig. 3 are different in the form of containing the anticoagulant. That is, in the blood collection container 1 illustrated in Fig. 1, the anticoagulant is contained in the blood collection container main body 2 in a state of being dissolved in a liquid, whereas in the blood collection container 1B illustrated in Fig. 3, the anticoagulant is contained on an inner wall surface of the blood collection container main body 2 in a powder state. In the blood collection container 1B illustrated in Fig. 3, the anticoagulant 5B is disposed in a layer.

(Method for separating mononuclear cells)

The mononuclear cells can be separated from the blood using the blood collection container. The method for separating mononuclear cells preferably includes a step of collecting blood in the blood collection container, and preferably includes a step of centrifuging the blood collection container from which the blood has been collected.

The centrifugation conditions in the centrifugation step are not particularly limited. Examples of the centrifugal separation condition include a condition of performing centrifugal separation at 400 G or more and 4000 G or less for 5 minutes or more and 120 minutes or less.

After the centrifugation step, for example, the red blood cell component is positioned below the partition wall formed by the blood separation material, the specific gravity liquid layer containing mononuclear cells is positioned on the partition wall formed by the blood separation material, and the plasma is positioned above the specific gravity liquid layer containing mononuclear cells. Mononuclear cells can be obtained by recovering the specific gravity liquid layer containing mononuclear cells.

The method for separating mononuclear cells is preferably a method for separating peripheral blood mononuclear cells (PBMC).

Hereinafter, the present invention will be specifically described with reference to examples and comparative examples. The present invention is not limited only to the following examples.

As a material of the specific gravity liquid, the following materials were prepared.

(Component (X))

Polysucrose ("Ficoll™PM 400" manufactured by Cytiva) Sodium diatrizoate
Silica fine particles ("Percoll" manufactured by Cytiva)
Sodium chloride

(Amino acid)

β-Alanine
Histidine
Proline
Glycine

Water

As materials of the composition for blood separation, the following were prepared.

(Material of organic component having fluidity at 25°C)

(Meth)acrylic resin:

2-ethylhexyl acrylate and butyl acrylate were radically polymerized in the presence of an azo polymerization initiator by a solution polymerization method to obtain a (meth)acrylic acid ester-based polymer having fluidity at 25°C. The specific gravity of the (meth)acrylic resin at 25°C was 1.033.

(Inorganic fine powder)

Hydrophilic silica (Fine powder silica, "200CF" manufactured by Nippon Aerosil Co., Ltd.)
Hydrophobic silica (Fine powder silica, "RX200" manufactured by Nippon Aerosil Co., Ltd.)
Calcium carbonate powder ("Socal UP-G" manufactured by IMERYS)

(Other components)

Silicone oil ("SF8410" manufactured by Dow Corning Toray Co., Ltd.)

Preparation of composition for blood separation:

91.9 parts by weight of a (meth)acrylic resin, 1.00 parts by weight of hydrophilic silica, 5.0 parts by weight of hydrophobic silica, 1.95 parts by weight of calcium carbonate powder, and 0.15 parts by weight of silicone oil were mixed to prepare a composition for blood separation. The specific gravity of the composition for blood separation at 25°C was 1.077.

(Example 1)

Preparation of specific gravity liquid:

A specific gravity liquid was obtained by blending and mixing the components shown in Table 1 in the blending proportions shown in Table 1. The blending amount in Table 1 and Tables 2 to 5 described later is a pure amount.

Preparation of blood collection container:

As a blood collection container main body, a polyethylene terephthalate container (PET bottomed tube) having a length of 100 mm and an opening end with an inner diameter of 14 mm was prepared. 1.0 mL of the obtained specific gravity liquid was contained in the blood collection container main body. Next, 1 mL of the obtained composition for blood separation was contained in a blood collection container main body. Subsequently, 30 mg of a 24 wt% aqueous solution of EDTA2K dihydrate (anticoagulant-containing solution) was applied onto the inner wall surface of the blood collection container main body and dried. Next, the inside of the blood collection container was decompressed so that the blood collection amount was 4 mL, and sealed with a butyl rubber stopper. In this way, a blood collection container in which the specific gravity liquid, the composition for blood separation, and the anticoagulant were contained in the blood collection container main body was produced. Subsequently, the blood collection container was irradiated with $\gamma$-rays at a dose of 30 kGy to obtain a blood collection container sterilized with $\gamma$-rays. In the obtained blood collection container, in the blood collection container main body, the specific gravity liquid is contained in the blood collection container main body closer to the bottom side than the composition for blood separation, and the composition for blood separation is contained in the blood collection container main body closer to the open end side than the specific gravity liquid. The anticoagulant is disposed on the inner wall surface on the open end side of the blood collection container main body with respect to the composition for blood separation.

(Examples 2 to 8 and Comparative Examples 1 to 5)

A specific gravity liquid and a blood collection container sterilized with $\gamma$ rays was manufactured in the same manner as in Example 1 except that the formulation composition of the specific gravity liquid was changed as shown in Tables 1 to 5.

(Evaluation)

(1) Specific gravity of specific gravity liquid

The specific gravity of the obtained specific gravity liquid at 25°C was measured using a specific gravity meter ("DA-130N" manufactured by Kyoto Electronics Manufacturing Co., Ltd.).

(2) Osmotic pressure of specific gravity liquid

The osmotic pressure of the obtained specific gravity liquid was measured by a freezing point depression method using an osmotic pressure meter ("OM-6060" manufactured by Arkray).

(3) Presence or absence of precipitate in specific gravity liquid

The specific gravity liquid in the blood collection container sterilized with $\gamma$ rays was visually observed to confirm whether or not precipitate was generated in the specific gravity liquid. The presence or absence of the precipitates in the specific gravity liquid was evaluated according to the following criteria. No precipitate was generated in the specific gravity liquid before being sterilized with $\gamma$ rays.

<Criteria for determination in presence or absence of precipitates in specific gravity liquid>

    A: No precipitate is generated in the specific gravity liquid
    B: Precipitates are generated in the specific gravity liquid

(4) Recovery amount of mononuclear cells (PBMC)

Blood of two persons (Donor A, Donor B) was prepared, and the following operations were performed for each of them. In the obtained blood collection container (blood collection container sterilized with $\gamma$ rays), 4 mL of blood was collected. The blood collection container from which the blood was collected was then centrifuged at 20°C and 1700 x g for 20 minutes. After centrifugation, the red blood cell layer was located below a partition wall formed by the composition for blood separation. In addition, the specific gravity liquid layer was positioned above the partition wall formed of the composition for blood separation, and a plasma layer was positioned above the specific gravity liquid layer. In addition, mononuclear cells were floating in the specific gravity liquid layer.

The specific gravity liquid layer was sucked up with a pipette and transferred to a centrifuge tube. Then, 1 mL of phosphate buffered saline was added to the centrifuge tube. The centrifuge tubes were then centrifuged at room temperature and 500 x g for 10 minutes to precipitate the cells. After removing the supernatant, the precipitated cells were suspended in 1 mL of plasma to obtain a suspension of mononuclear cells. The number of mononuclear cells in the suspension of mononuclear cells was measured by analyzing the suspension of mononuclear cells using a multi-item automated blood cell analyzer ("XE-5000" manufactured by Sysmex Corporation), and the number of mononuclear cells was defined as the recovery amount of mononuclear cells. In addition, the average of the recovery amount of mononuclear cells from the donor A and the recovery amount of mononuclear cells from the donor B was taken as the average recovery amount (Ave).

The increase amount ($\Delta$Ave) in the recovery amount of mononuclear cells due to the presence or absence of amino acid in the specific gravity liquid was determined between blood collection containers provided with specific gravity liquids having the same type and amount of the component (X). That is, the increase amount ($\Delta$Ave) is a value obtained by subtracting the average recovery amount (Ave) in the blood collection container as a reference from the average recovery amount (Ave) in the blood collection container to be evaluated. More specifically, the increase amount ($\Delta$Ave) is a value obtained by the following formula. The combination of the blood collection container to be evaluated and the reference blood collection container is as follows.

$\Delta$Ave = [Average recovery amount (Ave) in blood collection container to be evaluated] - [Average recovery amount (Ave) in blood collection container as reference]

More specifically, it is as follows.

$\Delta$Ave = [Average recovery amount (Ave) in blood collection container of Example 1] - [Average recovery amount (Ave) in blood collection container of Comparative Example 1]

$\Delta$Ave = [Average recovery amount (Ave) in blood collection container of Examples 2 to 5] - [Average recovery amount (Ave) in blood collection container of Comparative Example 2] ΔAve = [Average recovery amount (Ave) in blood collection container of Example 6] - [Average recovery amount (Ave) in blood collection container of Comparative Example 3] ΔAve = [Average recovery amount (Ave) in blood collection container of Example 7] - [Average recovery amount (Ave) in blood collection container of Comparative Example 4] ΔAve = [Average recovery amount (Ave) in blood collection container of Example 8] - [Average recovery amount (Ave) in blood collection container of Comparative Example 5]

<Criteria for determination in recovery amount of mononuclear cells>

A: ΔAve is 500,000 or more
B: ΔAve is more than 200,000 and less than 500,000
C: ΔAve is 200,000 or less

The configurations and results are shown in Tables 1 to 5 below.

[Table 1]

| | | | | Comparative Example 1 | Example 1 |
|---|---|---|---|---|---|
| Specific gravity liquid | Component (X) | Polysucrose | wt% | 5.8 | 5.8 |
| | | Sodium diatrizoate | wt% | 11.0 | 11.0 |
| | | Silica fine particles | wt% | | |
| | | Sodium chloride | wt% | | |
| | Amino acid | β-Alanine | wt% | | 0.25 |
| | | Histidine | wt% | | |
| | | Proline | wt% | | |
| | | Glycine | wt% | | |
| | Water | | wt% | 83.20 | 82.95 |
| | Total | | wt% | 100 | 100 |
| | Weight ratio (content of amino acid/content of component (X)) | | - | 0.00 | 0.01 |
| Specific gravity of specific gravity liquid | | | - | 1.089 | 1.089 |
| Osmotic pressure of specific gravity liquid | | | mOsm/L | 348 | 383 |
| Presence or absence of precipitate in specific gravity liquid | | | - | B | A |
| Recovery amount of mononuclear cells (PBMC) | Donor A | | Number of pieces | 100000 | 500000 |
| | Donor B | | Number of pieces | 300000 | 600000 |
| | Average recovery amount (Ave) | | Number of pieces | 200000 | 550000 |
| | Increase amount (ΔAve) | | Number of pieces | Reference | 350000 |
| | Determination | | - | | B |

[Table 2]

| | | | | Comparative Example 2 | Example 2 | Example 3 | Example 4 | Example 5 |
|---|---|---|---|---|---|---|---|---|
| Specific gravity liquid | Component (X) | Polysucrose | wt% | 10.6 | 10.6 | 10.6 | 10.6 | 10.6 |
| | | Sodium diatrizoate | wt% | 8.0 | 8.0 | 8.0 | 8.0 | 8.0 |
| | | Silica fine particles | wt% | | | | | |
| | | Sodium chloride | wt% | | | | | |
| | Amino acid | β-Alanine | wt% | | 0.25 | 0.40 | | |
| | | Histidine | wt% | | | | 1.00 | 0.50 |
| | | Proline | wt% | | | | | |
| | | Glycine | wt% | | | | | |
| | Water | | wt% | 81.40 | 81.15 | 81.00 | 80.40 | 80. 90 |
| | Total | | wt% | 100 | 100 | 100 | 100 | 100 |
| | Weight ratio (content of amino acid/content of component (X)) | | - | 0.00 | 0.01 | 0.02 | 0.05 | 0.03 |
| Specific gravity of specific gravity liquid | | | - | 1.088 | 1.088 | 1.089 | 1.092 | 1.090 |
| Osmotic pressure of specific gravity liquid | | | mOsm/L | 282 | 320 | 342 | 363 | 323 |
| Presence or absence of precipitate in specific gravity liquid | | | - | B | A | A | A | A |
| Recovery amount of mononuclear cells (PBMC) | Donor A | | Number of pieces | 4000000 | 5500000 | 5586000 | 4600000 | 5100000 |
| | Donor B | | Number of pieces | 3100000 | 4400000 | 5940000 | 5300000 | 6000000 |
| | Average recovery amount (Ave) | | Number of pieces | 3550000 | 4950000 | 5763000 | 4950000 | 5550000 |
| | Increase amount (ΔAve) | | Number of pieces | Reference | 1400000 | 2213000 | 1400000 | 2000000 |
| | Determination | | - | | A | A | A | A |

**EP 4 692 793 A1**

[Table 3]

| | | | | Comparative Example 3 | Example 6 |
|---|---|---|---|---|---|
| Specific gravity liquid | Component (X) | Polysucrose | wt% | 8.0 | 8.0 |
| | | Sodium diatrizoate | wt% | 9.5 | 9.5 |
| | | Silica fine particles | wt% | | |
| | | Sodium chloride | wt% | | |
| | Amino acid | β-Alanine | wt% | | 0.25 |
| | | Histidine | wt% | | |
| | | Proline | wt% | | |
| | | Glycine | wt% | | |
| | Water | | wt% | 82.50 | 82.25 |
| | Total | | wt% | 100 | 100 |
| | Weight ratio (content of amino acid/content of component (X)) | | - | 0.00 | 0.01 |
| Specific gravity of specific gravity liquid | | | - | 1.088 | 1.088 |
| Osmotic pressure of specific gravity liquid | | | mOsm/L | 310 | 352 |
| Presence or absence of precipitate in specific gravity liquid | | | - | B | A |
| Recovery amount of mononuclear cells (PBMC) | Donor A | | Number of pieces | 1400000 | 2300000 |
| | Donor B | | Number of pieces | 1100000 | 3000000 |
| | Average recovery amount (Ave) | | Number of pieces | 1250000 | 2650000 |
| | Increase amount (ΔAve) | | Number of pieces | Reference | 1400000 |
| | Determination | | - | | A |

[Table 4]

| | | | | Comparative Example 4 | Example 7 |
|---|---|---|---|---|---|
| Specific gravity liquid | Component (X) | Polysucrose | wt% | | |
| | | Sodium diatrizoate | wt% | | |
| | | Silica fine particles | wt% | 15.1 | 15.1 |
| | | Sodium chloride | wt% | 0.8 | 0.8 |
| | Amino acid | β-Alanine | wt% | | |
| | | Histidine | wt% | | |
| | | Proline | wt% | | 0.85 |
| | | Glycine | wt% | | |
| | Water | | wt% | 84.12 | 83.27 |
| | Total | | wt% | 100 | 100 |
| | Weight ratio (content of amino acid/content of component (X)) | | - | 0.00 | 0.05 |
| Specific gravity of specific gravity liquid | | | - | 1.088 | 1.090 |
| Osmotic pressure of specific gravity liquid | | | mOsm/L | 292 | 378 |
| Presence or absence of precipitate in specific gravity liquid | | | - | B | A |

(continued)

|  |  |  | Comparative Example 4 | Example 7 |
|---|---|---|---|---|
| Recovery amount of mononuclear cells (PBMC) | Donor A | Number of pieces | 0 | 400000 |
| | Donor B | Number of pieces | 0 | 450000 |
| | Average recovery amount (Ave) | Number of pieces | 0 | 425000 |
| | Increase amount (ΔAve) | Number of pieces | Reference | 425000 |
| | Determination | - | | B |

[Table 5]

|  |  |  |  | Comparative Example 5 | Example 8 |
|---|---|---|---|---|---|
| Specific gravity liquid | Component (X) | Polysucrose | wt% | 10.6 | 10.6 |
| | | Sodium diatrizoate | wt% | 8.0 | 8.0 |
| | | Silica fine particles | wt% | | |
| | | Sodium chloride | wt% | | |
| | Amino acid | β-Alanine | wt% | | |
| | | Histidine | wt% | | |
| | | Proline | wt% | | |
| | | Glycine | wt% | | 0.40 |
| | Water | | wt% | 81.40 | 81.00 |
| | Total | | wt% | 100 | 100 |
| | Weight ratio (content of amino acid/content of component (X)) | | - | 0.00 | 0.02 |
| Specific gravity of specific gravity liquid | | | - | 1.088 | 1.091 |
| Osmotic pressure of specific gravity liquid | | | mOsm/L | 282 | 335 |
| Presence or absence of precipitate in specific gravity liquid | | | - | B | A |
| Recovery amount of mononuclear cells (PBMC) | Donor A | | Number of pieces | 3500000 | 6200000 |
| | Donor B | | Number of pieces | 4200000 | 5500000 |
| | Average recovery amount (Ave) | | Number of pieces | 3850000 | 5850000 |
| | Increase amount (ΔAve) | | Number of pieces | Reference | 2000000 |
| | Determination | | - | | A |

**EXPLANATION OF SYMBOLS**

1, 1A, 1B: Blood collection container
2: Blood collection container main body
2a: Open end
2b: Bottom
3: Specific gravity liquid
4: Composition for blood separation
5: Liquid containing anticoagulant
5A, 5B: Powdery anticoagulant
6: Plug

**Claims**

1. A blood collection container comprising:

   a blood collection container main body;
   a specific gravity liquid contained in the blood collection container main body; and
   a blood separation material contained in the blood collection container main body,
   the specific gravity liquid containing an amino acid.

2. The blood collection container according to claim 1, wherein the amino acid contains a basic amino acid or a neutral amino acid.

3. The blood collection container according to claim 1 or 2, wherein the amino acid contains β-alanine, proline, histidine, or glycine.

4. The blood collection container according to any one of claims 1 to 3, wherein the amino acid contains β-alanine.

5. The blood collection container according to any one of claims 1 to 4, wherein a content of the amino acid is 0.01 wt% or more and 5 wt% or less in 100 wt% of the specific gravity liquid.

6. The blood collection container according to any one of claims 1 to 5, wherein the specific gravity liquid contains a component (X) that is solid at 25°C different from the amino acid.

7. The blood collection container according to claim 6, wherein the component (X) contains polysucrose.

8. The blood collection container according to claim 6, wherein the component (X) contains sodium diatrizoate.

9. The blood collection container according to claim 6, wherein the component (X) contains polysucrose and sodium diatrizoate.

10. The blood collection container according to any one of claims 6 to 9, wherein the component (X) contains silica fine particles.

11. The blood collection container according to any one of claims 6 to 10, wherein a weight ratio of the content of the amino acid to the content of the component (X) in the specific gravity liquid is 0.001 or more and 0.5 or less.

12. The blood collection container according to any one of claims 1 to 11, wherein the blood separation material is a composition for blood separation.

13. The blood collection container according to claim 12, wherein the composition for blood separation contains an organic component having fluidity at 25°C and an inorganic fine powder,

   the organic component contains a resin, and
   the inorganic fine powder contains fine powder silica.

14. The blood collection container according to any one of claims 1 to 13, comprising an anticoagulant contained in the blood collection container main body.

15. The blood collection container according to any one of claims 1 to 14, wherein the blood collection container main body is a polyethylene terephthalate container.

16. The blood collection container according to any one of claims 1 to 15, which is a blood collection container sterilized with γ rays.

17. A method for separating mononuclear cells, comprising the steps of:

   collecting blood into the blood collection container according to any one of claims 1 to 16; and
   centrifuging the blood collection container into which the blood has been collected.

[FIG. 1.]

1

6

2a

2

5

4

3

2b

[FIG. 2.]

[FIG. 3.]

## INTERNATIONAL SEARCH REPORT

| International application No. |
| --- |
| **PCT/JP2024/012963** |

| A. | CLASSIFICATION OF SUBJECT MATTER |
| --- | --- |

*G01N 33/48*(2006.01)i
FI:   G01N33/48 D

According to International Patent Classification (IPC) or to both national classification and IPC

| B. | FIELDS SEARCHED |
| --- | --- |

Minimum documentation searched (classification system followed by classification symbols)

G01N33/48: A61B5/15

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 61-84557 A (BECTON DICKINSON & CO.) 30 April 1986 (1986-04-30) entire text, all drawings | 1-17 |
| A | JP 8-224227 A (INTERNATL. REAGENTS CORP.) 03 September 1996 (1996-09-03) entire text, all drawings | 1-17 |
| A | JP 2002-365280 A (SEKISUI CHEMICAL CO., LTD.) 18 December 2002 (2002-12-18) entire text, all drawings | 1-17 |
| A | WO 2006/098350 A1 (SEKISUI CHEMICAL CO., LTD.) 21 September 2006 (2006-09-21) entire text, all drawings | 1-17 |
| A | JP 9-196910 A (FUJI PHOTO FILM CO., LTD.) 31 July 1997 (1997-07-31) entire text, all drawings | 1-17 |

☐ Further documents are listed in the continuation of Box C.   ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **14 May 2024** | **28 May 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

INTERNATIONAL SEARCH REPORT
Information on patent family members

International application No.

**PCT/JP2024/012963**

| Patent document cited in search report | | | Publication date (day/month/year) | Patent family member(s) | | | Publication date (day/month/year) |
|---|---|---|---|---|---|---|---|
| JP | 61-84557 | A | 30 April 1986 | US whole document EP | 4751001 176080 | A A2 | |
| JP | 8-224227 | A | 03 September 1996 | (Family: none) | | | |
| JP | 2002-365280 | A | 18 December 2002 | (Family: none) | | | |
| WO | 2006/098350 | A1 | 21 September 2006 | US whole document EP CN KR | 2008/0274532 1860436 101137902 10-2007-0111441 | A1 A1 A A | |
| JP | 9-196910 | A | 31 July 1997 | (Family: none) | | | |

Form PCT/ISA/210 (patent family annex) (July 2022)

**REFERENCES CITED IN THE DESCRIPTION**

**Patent documents cited in the description**

- JP S6184557 A

- WO 2010132783 A1